Europäisches Patentamt

**European Patent Office**

Office européen des brevets

(19)

(11) Publication number: **0 064 701**
**B1**

(12) **EUROPEAN PATENT SPECIFICATION**

(45) Date of publication of patent specification: **09.07.86**

(51) Int. Cl.⁴: **A 61 M 16/00**

(21) Application number: **82103732.2**

(22) Date of filing: **01.05.82**

(54) Endotracheal tube with movable endobronchial blocker.

(30) Priority: **07.05.81 JP 68698/81**

(43) Date of publication of application:
**17.11.82 Bulletin 82/46**

(45) Publication of the grant of the patent:
**09.07.86 Bulletin 86/28**

(84) Designated Contracting States:
**DE FR GB**

(56) References cited:
**DE-A-2 711 977**
**FR-A-1 505 607**
**GB-A-2 003 038**
**US-A-3 417 744**

(73) Proprietor: **Inoue, Hiroshi**
**No. 15-20, Shiratori 1-chome Tama-ku**
**Kawasaki Prefecture of Kanagawa (JP)**

(72) Inventor: **Inoue, Hiroshi**
**No. 15-20, Shiratori 1-chome Tama-ku**
**Kawasaki Prefecture of Kanagawa (JP)**

(74) Representative: **Schulze Horn, Stefan, Dipl.-Ing.**
**et al**
**Patentanwälte Dipl.-Ing. S. Schulze Horn M.SC.**
**Dr. H. Hoffmeister Goldstrasse 36**
**D-4400 Münster (DE)**

Courier Press, Leamington Spa, England.

## Description

The present invention refers to an apparatus to be used for carrying out one-lung anesthesia, comprising
—an endotracheal tube with an inflatable cuff, mounted on the outer periphery of said tube near the forward end thereof, said tube forming a ventilation duct, and
—an endotracheal blocker consisting of a catheter with a second inflatable cuff, mounted on the periphery near the forward end thereof.

Operations for lung cancer, esophegeal cancer, thoracic aorta, etc. that need a thoracotomy are often disturbed by lung ventilation. Keeping such a lung depressed with a retractor may damage the lung tissue. On the other hand, it has been confirmed by many researches, in the case of a patient who is certain to stand the aforesaid operations judging from his cardiopulmonary function, there is not any anesthetic trouble and danger during operation, even if the contralateral lung alone is ventilated, in other words even if the operated lung is completely collapsed. This technique is named "one-lung anesthesia". One-lung anesthesia can be of great assistance to the surgeon not only during the operation on the lung but also operations on the oesophagus, mediastinum and thoracic aorta.

Known in the art is an apparatus to be used for carrying out one-lung anesthesia (FR—A—1.505.607), which comprises an outer tube, which contains a catheter with a cuff ("blocker") in the lumen of said endotracheal tube. This apparatus has serious drawbacks.

With the known apparatus one-lung anesthesia is performed by inserting a small diametral endobronchial tube into the lumen of a large diametral endotracheal tube and leaving it in the main bronchus. Therefore it will be possible to perform one-lung anesthesia by virtue of the small diametral endobronchial tube. It cannot be seen, however, how the artificial anesthesia of the other lung may be performed when the tube is used as a blocker.

Further the known apparatus is a two-tube system with only small space left between the surfaces of the tubes. The inner movable tube or blocker carries a cuff at its lower end, said cuff to be positioned in one of the main bronchi. The flexibility of said combination of two tubes is very low, thus making it difficult to position the catheter in the desired position or branch of the bronchus. The necessary movements also bear the risk of hurting the inner surface of said organs.

It is therefore the object of the invention to provide an apparatus of the kind mentioned above, which has a large opening for ventilation and can be assembled into a highly flexible system which can easily be used for the right or left bronchus without the hazard of hurting the inner surface of trachea and bronchus, and can perform the anesthesia of the other lung, where the small tube is used as a blocker.

This object is accomplished according to the invention with an apparatus of the kind mentioned above, whereby said catheter is movably received in a small channel formed within the wall of the endotracheal tube along almost the entire length of said tube, said channel and the ventilation duct of the endotracheal tube being separated by a partition wall extending to, or nearly to, said forward end (35a) of said endotracheal tube (35). The present invention not only overcomes the drawbacks mentioned above, but also makes it possible to shift to bilateral ventilation simply by vacuation of the cuff which is blocking the bronchus.

Further features of the invention, mentioned in the subclaims, will be discussed with the description of the drawings. The Figures of the drawings are showing:

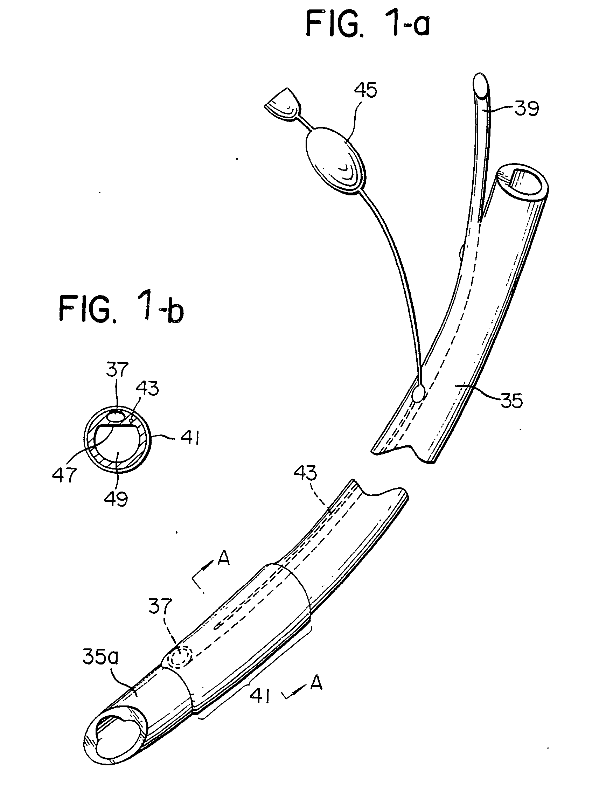

Fig. 1-a is a perspective view of the endotracheal tube of this invention.

Fig. 1-b is a sectional view taken along line A—A in Fig. 1-a.

Fig. 2-a is a perspective view of the endobronchial blocker of the invention.

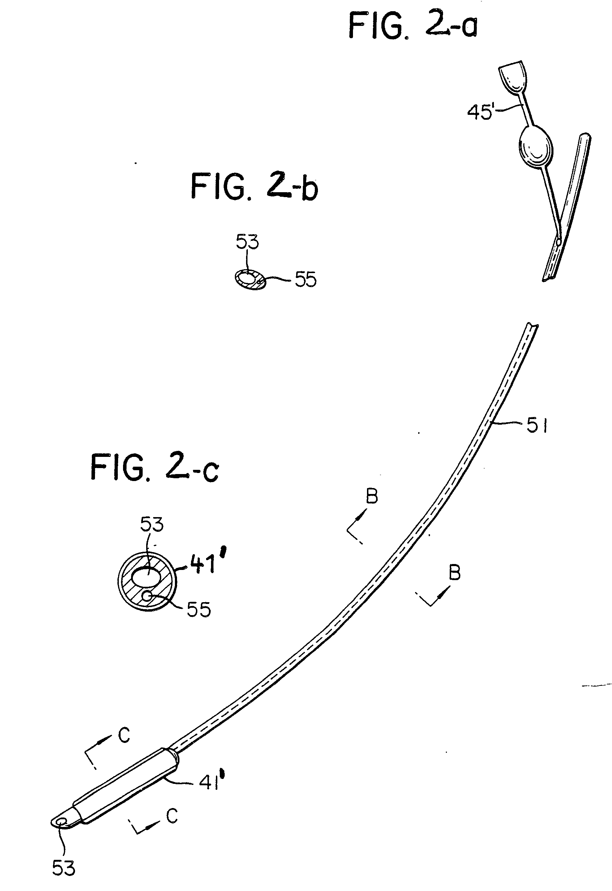

Fig. 2-b and Fig. 2-c are sectional views taken along lines B—B and C—C, respectively, in Fig. 2-a.

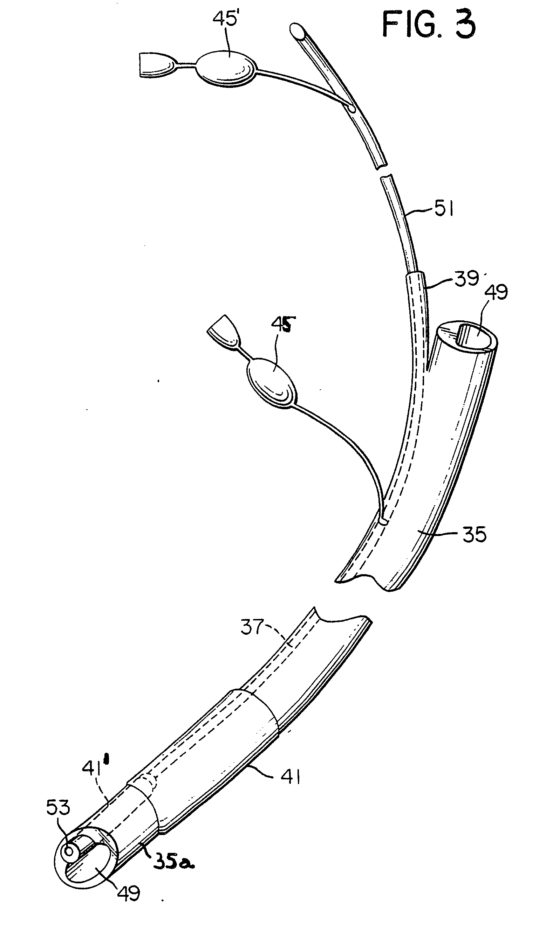

Fig. 3 is a perspective view of the endotracheal tube with movable endobronchial blocker for one-lung anesthesia of this invention.

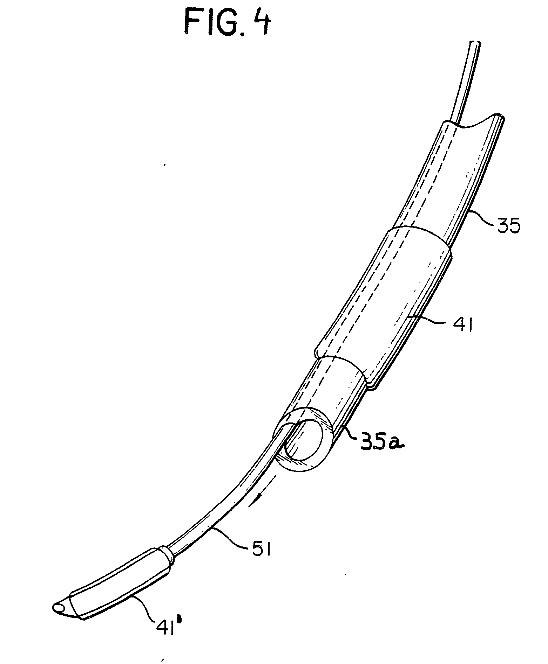

Fig. 4 is a perspective view showing the endobronchial blocker which is being let out from the small channel in the endotracheal tube.

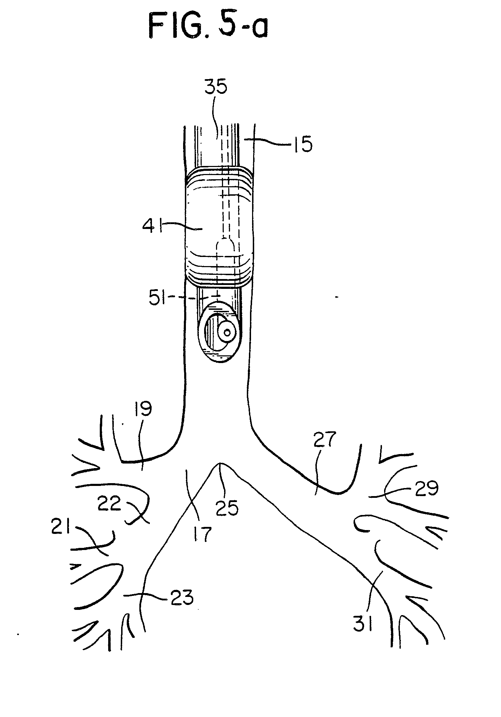

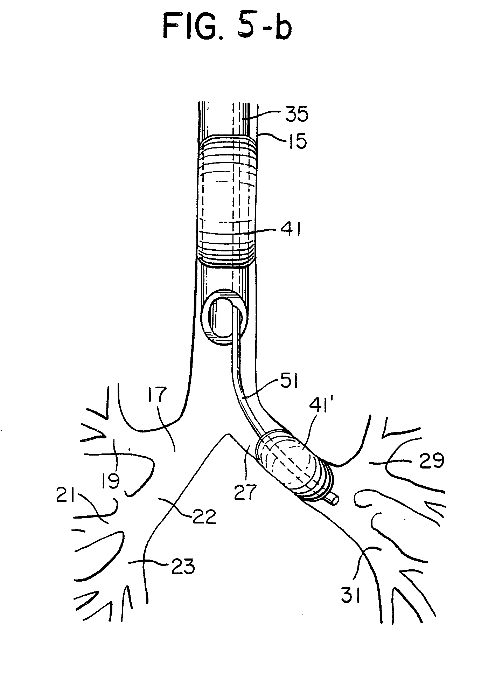

Fig. 5-a and 5-b illustrate one-lung anesthesia for the right lung being performed by using the endotracheal tube with movable endobronchial blocker for one-lung anesthesia of this invention.

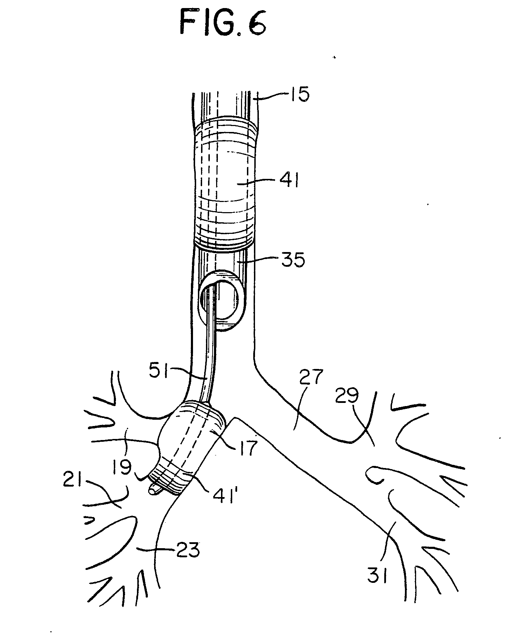

Fig. 6 illustrates one-lung anesthesia for the left lung being performed by using the endotracheal tube with movable endobronchial blocker for one-lung anesthesia of this invention.

Fig. 1-a is a perspective view of an example of an endotracheal tube belonging to an apparatus for one-lung anesthesia according to the invention. Fig. 1-b is a sectional view along the line A—A in Fig. 1-a. The tube may be made of silicone or the like.

The endotracheal tube 35 is similar to a conventional endotracheal tube in external appearance except that it has a branching guide tube 39 which communicates with a small channel 37 showing about 3 cm in the direction toward the upper opening of the tube 35. It has, for example, an inside diameter of about 3.5 mm or an oval section of 3 mm by 4 mm. The tube 35 has a cuff 41 near the end 35a thereof, and the cuff 41 communicates with an air blowing means 45 through an air supply duct 43 for cuff inflation. The small channel 37 extending along almost the entire length of the tube is formed in the concavely curved wall of the tube by simultaneous molding or bonding. Therefore, the ventilation duct 49 is adjacent to the small channel 37, but is separated therefrom by partition wall 47 in the section as shown in Fig. 1-b.

Several centimeters away from the end 35a of the endotracheal tube 35, the partition wall 47 separating the small channel 37 from the ventilation duct 49 may be omitted as shown in Fig. 1-a. In such a structure, the small channel 37 may have the same tunnel-like shape as the branching guide tube 39. In this tunnel-like channel 37 and branching guide tube 39 the catheter part 51 of the endobronchial blocker 51+41' is housed. In the forward end 35a of the endotracheal tube 35 where there is no partition wall 47 the cuff 41' of the catheter 51 is placed. Consequently, the part near the forward end 35a of the endobronchial blocker 51+41' can be shaped differently. Needless to say, it is also possible to provide the small channel 37 up to the forward end 35a of the endotracheal tube by forming the partition wall 47 as far as the forward end 35a of the endotracheal tube 35. The branching guide tube 39 is intended to facilitate the manipulation of the endobronchial blocker 51+41'.

Fig. 2-a shows the endobronchial blocker 51+41' which is inserted and housed in the small channel 37 of the endotracheal tube 35 as shown in Fig. 1. As illustrated in Fig. 2-b, the catheter part 51 of the endobronchial blocker 51+41' (about 40 cm long) has the duct 53 for aspiration of gas or bronchial secretion from the blocked bronchus. Note the small duct 55 for inflation of the cuff 41' of the endobronchial blocker 51+41' by the cuff inflating means 45'. The duct 53 may be omitted depending on the usage. As shown in the sectional view of Fig. 2-c, the forward end of the catheter 51 of the endobronchial blocker 51+41' to which the low-pressure high-volume cuff 41' (about 3.5 cm long) is attached may be larger in diameter (about 5 mm) and have a circular cross section. By forcing air into this part, the main bronchus can be blocked with the cuff 41'. Needless to say that it is possible to make the diameter of the catheter 51 of the endobronchial blocker 51+41' substantially the same throughout the entire length. It is desirable that the endobronchial blocker 51+41' is slightly curved over the entire length. As shown in Fig. 3, the endobronchial blocker 51+41' is housed initially in the small channel 37 of the endotracheal tube 35 in such a manner that it can be moved smoothly in the channel 37. When not in use, they may be stored separately.

Fig. 4 shows the relative position of the endotracheal tube 35 of this invention and the endobronchial blocker 51+41' in use. The endobronchial blocker 51+41' is let out from the forward end 35a of the endotracheal tube 35 so that the endobronchial blocker 51+41' is inserted into the targeted main bronchus. The bronchus of the operated lung is then ready to be blocked.

The endotracheal tube 35 of this invention can be used in the following manner. A shown in Fig. 5-a and Fig. 5-b, at first, the endotracheal tube 35 with movable endobronchial blocker 51+41' for one-lung anesthesia is inserted into the trachea 15 of a patient by a usual method. If right one-lung anesthesia is to be performed during operations, the inserted endotracheal tube 35 is turned (axially) counterclockwise so that the endobronchial blocker 51+41' is directed to the left and the cuff 41 of the endotracheal tube 35 is inflated to fix the tube 35 in position (Fig. 5-a). Then, the endobronchial blocker 51+41' is let out as far as the marked position (about 6 cm). Since the endobronchial blocker 51+41' is formed so that it is curved outward, the forward end of the endobronchial blocker 51+41', is moved forward while being kept in contact with the left wall of the trachea 15. Thus, the forward end of the endobronchial blocker 51+41' is inserted easily and certainly into the left bronchus 27, and the left bronchus 27 is blocked with certainty by the cuff 41' (Fig. 5-b).

On the other hand, if the left one-lung anesthesia is to be performed during operations, the inserted endotracheal tube 35 is turned (axially) clockwise so that the endobronchial blocker 51+41' is directed and inserted into the right bronchus 17 as in above and the cuff 41' is expanded, as shown in Fig. 6.

Whether or not the endobronchial blocker has been inserted correctly into bronchus of the targeted side may be confirmed by the aid of chest roentgenogram or flexible bronchofiberscope. Needless to say that a skilled chest surgeon will be able to determine readily the position of the cuff of the endobronchial blocker by palpation of the bronchus.

In case any trouble should occur during anesthesia by unilateral ventilation or one-lung anesthesia with the tube of this invention, it is possible to readily shift to bilateral ventilation simply by evacuation of the cuff which is blocking the bronchus.

## Claims

1. An apparatus to be used for carrying out one-lung anesthesia, comprising
—an endotracheal tube (35) with an inflatable cuff (41), mounted on the outer periphery of said tube near the forward end (35a) thereof, said tube forming a ventilation duct (49), and
—an endobronchial blocker (51+41') consisting of a catheter (51) with a second inflatable cuff (41'), mounted on the outer periphery near the forward end thereof, characterized by that
said catheter (51) is movably received in a small channel (37) formed within the wall of the endotracheal tube (35) along almost the entire length of said tube (35), said channel (37) and the ventilation duct (49) of the endotracheal tube being separated by a partition wall (47) extending to, or nearly to, said forward end (35a) of said endotracheal tube (35).

2. An apparatus according to claim 1, characterized by that the endotracheal tube (35) is curved, and the small channel (37) is formed on the concave side of the curve.

3. An apparatus according to claim 1 or 2, characterized by that the small channel (37) communicates with a branching guide tube (39) which branches out from said endotracheal tube (35), showing toward the rear end of said tube.

4. An apparatus according to claim 1, characterized in that when the partition wall (47) between the small channel (37) and the ventilation duct (49) ends before the forward end of said endotracheal tube, it opens a direct communication of the small channel (37) and the ventilation duct (49).

5. An apparatus according to one or more of the foregoing claims, characterized by that the catheter (51) is curved.

6. An apparatus according to claims 1—5, characterized by that an aspiration duct (53) is formed within said catheter with an opening at the vicinity of the forward end of the catheter (51).

7. An apparatus according to claims 1—6, characterized by that the diameter of the forward end of the catheter (51) is larger than the remainder of said catheter extending rearwardly therefrom.

## Patentansprüche

1. Vorrichtung zur Verwendung bei der Durchführung der Ein-Flügel-Lungen-Anästhesie, umfassend .

—einen Endotracheal-Tubus (35) mit einer am Außenumfang des Tubus nahe seinem Vorderende (35a) montierten, aufblasbaren Manschette (41) wobei der Tubus eine Belüftungsleitung (49) bildet, und

—eine Endobronchial-Abdichtung (51+41') bestehend aus einem Katheter (51) mit einer zweiten, am Außenumfang nahe seinem Vorderende angebrachten, aufblasbaren Manschette (41'), dadurch gekennzeichnet, daß der Katheter (51) verschiebbar in einem engen, in der Wand des Endotracheal-Tubus (35) über fast die Gesamtlänge des Tubus (35) ausgebildeten Durchgang (37) eingesetzt ist, wobei der Durchgang (37) und die Belüftungsleitung (49) des Endotracheal-Tubus durch eine Trennwand (47), die sich bis zum Vorderende (35a) des Endotracheal-Tubus (35) oder nahezu bis zu diesem Vorderende erstreckt, voneinander getrennt sind.

2. Vorrichtung nach Anspruch 1, dadurch gekennzeichnet, daß der Endotracheal-Tubus (35) gekrümmt ist und der enge Durchgang (37) an der konkaven Seite der Krümmung ausgebildet ist.

3. Vorrichtung nach Anspruch 1 oder 2, dadurch gekennzeichnet, daß der enge Durchgang (37) mit einem Abzweig-Leitrohr (39) kommuniziert, das, zum hinteren Ende des Tubus weisend, vom Endotracheal-Tubus (35) nach außen abzweigt.

4. Vorrichtung nach Anspruch 1, dadurch gekennzeichnet, daß dann wenn die Trennwand (47) zwischen dem engen Durchgang (37) und der Belüftungsleitung (49) vor dem Vorderende des Endotracheal-Tubus endet, diese Trennwand eine unmittelbare Verbindung zwischen dem engen Durchgang (37) und der Belüftungsleitung (49) herstellt.

5. Vorrichtung nach einem oder mehreren der vorangehenden Ansprüche, dudurch gekennzeichnet, daß der Katheter (51) gekrümmt ist.

6. Vorrichtung nach den Ansprüchen 1 bis 5, dadurch gekennzeichnet, daß im Katheter eine Aspirationsleitung (53) mit einer Mündung in der Nähe des Vorderendes des Katheters (51) ausgebildet ist.

7. Vorrichtung nach den Ansprüchen 1 bis 6, dadurch gekennzeichnet, daß der Durchmesser des Vorderendes des Katheters (51) größer ist als der des davon rückwärts abgehenden restlichen Teils des Katheters.

## Revendications

1. Un appareil destiné à être utilisé pour effectuer une anesthésie sur un seul poumon, comprenant:

—un tube endotrachéal (35) sur la périphérie extérieure duquel, vers l'extrémité antérieure (35a) dudit tube, est monté un manchon gonflable (41), ledit tube formant un conduit de ventilation (49); et

—un obturateur endotrachéal (51+41') consistant en un cathéter (51) avec un second manchon gonflable (41') monté sur la périphérie extérieure, vers l'extrémité antérieure, de ce cathéter, caractérisé en ce que ledit cathéter (51) est reçu de manière mobile dans un petit canal (37) ménagé dans la paroi du tube endotrachéal (35) suivant la presque totalité de la longueur dudit tube (35), ledit canal (37) et le conduit de ventilation (49) du tube endotrachéal étant séparés par une paroi de séparation (47) s'étendant jusqu'à ladite extrémité antérieure (35a) dudit tube endotrachéal (35) ou jusqu'à proximité immédiate de cette extrémité.

2. Un appareil selon la revendication 1, caractérisé en ce que le tube endotrachéal (35) est incurvé et en ce que le petit canal (37) est formé sur la face concave de l'incurvation.

3. Un appareil selon la revendication 1 ou 2, caractérisé en ce que le petit canal (37) est en communication avec un tube de connexion (39) qui se branche sur ledit tube endotrachéal (35) à partir de l'extrémité arrière dudit tube.

4. Un appareil selon la revendication 1, caractérisé en ce que, lorsque la paroi de séparation (47) entre le petit canal (37) et le conduit de ventilation (49) se termine avant l'extrémité arrière dudit tube endotrachéal, elle met en communication directe le petit canal (37) et le conduit de ventilation (49).

5. Un appareil selon une ou plusieurs des revendications précédentes, caractérisé en ce que le cathéter (51) est incurvé.

6. Un appareil selon les revendications 1 à 5, caractérisé en ce qu'un conduit d'aspiration (53), formé à l'intérieur dudit cathéter comporte une ouverture à proximité de l'extrémité arrière du cathéter (51).

7. Un appareil selon les revendications 1 à 6, caractérisé en ce que le diamètre de l'extrémité avant du cathéter (51) est supérieur à celui de la partie restante dudit cathéter se trouvant placée à l'arrière.

# FIG. 1-a

45

39

35

# FIG. 1-b

37

43

41

47

49

43

A

37

35a

A

41

# FIG. 2-a

45'

# FIG. 2-b

53

55

51

B

B

# FIG. 2-c

53

41'

55

C

41'

C

53

0 064 701

FIG. 3

# FIG. 4

35

41

35a

51

41'

**0 064 701**

# FIG. 5-a

# FIG. 5-b

0 064 701

FIG. 6